# EUROPÄISCHE PATENTANMELDUNG

(11) **EP 0 825 185 A1**
(43) Veröffentlichungstag der Anmeldung: **25.02.1998**
(21) Anmeldenummer: 97110360.1
(22) Anmeldetag: 25.06.1997
(51) Int. Cl.: C07D 221/16, A61K 31/44

(54) **Bicyclisch kondensierte Pyridine**

(30) Priorität: 08.07.1996 DE 19627430
(71) Anmelder: BAYER AG, 51368 Leverkusen (DE)
(72) Erfinder: Brandes, Arndt, Dr., 42115 Wuppertal (DE); Lögers, Michael, Dr., 42327 Wuppertal (DE); Schmidt, Gunter, Dr., 42115 Wuppertal (DE); Angerbauer, Rolf, Dr., Higashinada-ku, Kobe-shi, Hyogo 658 (JP); Schmeck, Carsten, Dr., 42113 Wuppertal (DE); Bremm, Klaus-Dieter, Dr., 45661 Recklinghausen (DE); Bischoff, Hilmar, Dr., 42113 Wuppertal (DE); Schmidt, Delf, Dr., 42113 Wuppertal (DE); Schuhmacher, Joachim, Dr., 42113 Wuppertal (DE)

(57) **Zusammenfassung**

Bicyclisch kondensierte Pyridine werden hergestellt, in dem man durch Umsetzung der enstprechenden Aldehyde mit Hilfe von Wittig- oder Grignardreagenzien die entsprechende Seitenkette einführt. Die bicyclisch kondensierten Pyridine eignen sich als Wirkstoffe in Arzneimitteln insbesondere in Arzneimitteln zur Behandlung von Hyperlipoproteinaemia und Arteriosklerose.

## Beschreibung

Die vorliegende Erfindung betrifft bicyclisch kondensierte Pyridine, Verfahren zu ihrer Herstellung und ihre Verwendung in Arzneimitteln.

Aus der Publikation US 5 169 857 A2 sind 7-(polysubstituierte Pyridyl)-6-heptenoate zur Behandlung der Arteriosklerose, Lipoproteinaemia und Hyperlipoproteinaemia bekannt. Außerdem wird die Herstellung von 7-(4-Aryl-3-pyridyl)-3,5-dihydroxy-6-heptenoate in der Publikation EP 325 130 A2 beschrieben. Ferner ist die Verbindung 3-Benzoyl-2-methyl-4-phenyl-indeno〈1,2-b〉pyridin-5-on aus der Publikation J. Chem. Soc. 1949, 2134, 2137 bekannt.

Die vorliegende Erfindung betrifft bicyclisch kondensierte Pyridine der allgemeinen Formel (I), 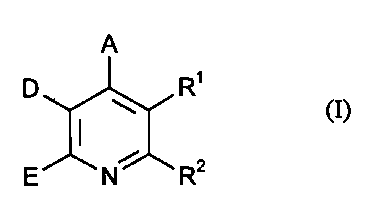 in welcher
- A: für Aryl mit 6 bis 10 Kohlenstoffatomen steht, das gegebenenfalls bis zu 5-fach gleich oder verschieden durch Halogen, Hydroxy, Trifluormethyl, Trifluormethoxy, Nitro oder durch geradkettiges oder verzweigtes Alkyl, Acyl, Hydroxyalkyl oder Alkoxy mit jeweils bis zu 7 Kohlenstoffatomen, oder durch eine Gruppe der Formel -NR³R⁴ substituiert ist,
worin
- R³ und R⁴: gleich oder verschieden sind und
Wasserstoff, Phenyl oder geradkettiges oder verzweigtes Alkyl mit bis zu 6 Kohlenstoffatomen bedeuten,
- D: für einen Rest der Formel 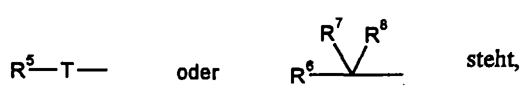 worin
- R⁵ und R⁶: unabhängig voneinander
Cycloalkyl mit 3 bis 8 Kohlenstoffatomen bedeuten, oder
Aryl mit 6 bis 10 Kohlenstoffatomen,
oder einen 5- bis 7-gliedrigen aromatischen, gegebenenfalls benzokondensierten Heterocyclus mit bis zu 3 Heteroatomen aus der Reihe S, N und/oder O bedeuten, die gegebenenfalls bis zu 5-fach gleich oder verschieden durch Trifluormethyl, Trifluormethoxy, Halogen, Hydroxy, Carboxyl, durch geradkettiges oder verzweigtes Alkyl, Acyl, Alkoxy oder Alkoxycarbonyl mit jeweils bis zu 6 Kohlenstoffatomen oder durch Phenyl, Phenoxy oder Thiophenyl substituiert sind, die ihrerseits durch Halogen, Trifluormethyl oder Trifluormethoxy substituiert sein können,
oder die Cyclen durch eine Gruppe der Formel -NR⁹R¹⁰ substituiert sind,
worin
- R⁹ und R¹⁰: gleich oder verschieden sind und die oben angegebene Bedeutung von R³ und R⁴ haben,
- T: eine geradkettige oder verzweigte Alkylen- oder Alkenylenkette mit jeweils bis zu 10 Kohlenstoffatomen bedeutet, die gegebenenfalls bis zu 2-fach durch Hydroxy substituiert sind,
- R⁷: Wasserstoff oder Halogen bedeutet,
und
- R⁸: Wasserstoff, Halogen, Azido, Trifluormethyl, Hydroxy, Trifluormethoxy, geradkettiges oder verzweigtes Alkoxy mit bis zu 5 Kohlenstoffatomen oder einen Rest der Formel -NR¹¹R¹² bedeutet,
worin
- R¹¹ und R¹²: gleich oder verschieden sind und die oben angegebene Bedeutung von R³ und R⁴ haben,
oder
- R⁷ und R⁸: gemeinsam mit dem C-Atom eine Carbonylgruppe bilden,
- E: für Cycloalkyl mit 3 bis 8 Kohlenstoffatomen steht, oder
für geradkettiges oder verzweigtes Alkyl mit bis zu 8 Kohlenstoffatomen steht, das gegebenenfalls durch Cycloalkyl mit 3 bis 8 Kohlenstoffatomen oder durch Hydroxy substituiert ist,
- R¹ und R²: gemeinsam eine geradkettige oder verzweigte Alkylenkette mit bis zu 8 Kohlenstoffatomen bilden, an die ein Phenylring anneliert ist und die durch eine Carbonylgruppe oder eine Gruppe der Formel 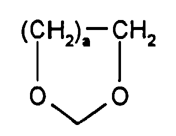 oder -OR¹³ substituiert sein muß,
worin
- a: für eine Zahl 1, 2 oder 3 steht,
und
- R¹³: Wasserstoff, geradkettiges oder verzweigtes Alkyl, Acyl oder Alkoxycarbonyl mit jeweils bis zu 6 Kohlenstoffatomen oder einen Rest der Formel -SiR¹⁴R¹⁵R¹⁶ bedeutet,
worin
- R¹⁴, R¹⁵ und R¹⁶: gleich oder verschieden sind und
geradkettiges oder verzweigtes Alkyl mit bis zu 6 Kohlenstoffatomen oder Phenyl bedeuten,
und wobei beide Ringsysteme gegebenenfalls bis zu 3-fach gleich oder verschieden durch Halogen, Carboxyl oder durch geradkettiges oder verzweigtes Alkyl, Alkoxy oder Alkoxycarbonyl mit jeweils bis zu 6 Kohlenstoffatomen substituiert sind,
und deren Salze und deren N-Oxide, mit Ausnahme der Verbindung 3-Benzoyl-2-methyl-4-phenyl-indeno〈1,2-b〉pyridin-5-on.

Die erfindungsgemäßen bicyclisch-substituierten Pyridine können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten oder ungesättigten 5- bis 7-gliedrigen, vorzugsweise 5- bis 6-gliedrigen Heterocyclus der bis zu 3 Heteroatome aus der Reihe S, N und/oder O enthalten kann. Beispielsweise seien genannt: Indolyl, Isochinolyl, Chinolyl, Benzo[b]thiophenyl, Benzo[b]furyl, Pyridyl, Thienyl, Furyl, Pyrrolyl, Thiazolyl, Oxazolyl, Imidazolyl, Morpholinyl oder Piperidyl. Bevorzugt sind Chinolyl, Furyl, Pyridyl und Thienyl.

Bevorzugt sind die erfindungsgemäßen Verbindungen der allgemeinen Formel (I),
in welcher
- A: für Naphthyl oder Phenyl steht, die gegebenenfalls bis zu 3-fach gleich oder verschieden durch Fluor, Chlor, Brom, Hydroxy, Trifluormethyl, Trifluormethoxy, Nitro oder durch geradkettiges oder verzweigtes Alkyl oder Alkoxy mit jeweils bis zu 6 Kohlenstoffatomen substituiert sind,
- D: für einen Rest der Formel 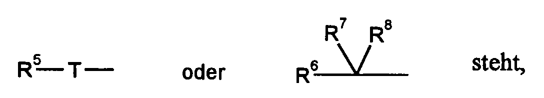 worin
- R⁵ und R⁶: unabhängig voneinander
Cyclopropyl, Cyclopentyl oder Cyclohexyl bedeuten, oder
Naphthyl, Phenyl, Pyridyl, Chinolyl, Indolyl, Benzthiazolyl oder Tetrahydronaphthalinyl bedeuten, die gegebenenfalls bis zu 3-fach gleich oder verschieden durch Trifluormethyl, Trifluormethoxy, Fluor, Chlor, Brom, Hydroxy, Carboxyl, durch geradkettiges oder verzweigtes Alkyl, Acyl, Alkoxy oder Alkoxycarbonyl mit jeweils bis zu 5 Kohlenstoffatomen oder durch Phenyl, Phenoxy oder Thiophenyl substituiert sind, die ihrerseits durch Fluor, Chlor, Brom, Trifluormethyl oder Trifluormethoxy substituiert sein können,
- T: eine geradkettige oder verzweigte Alkylen- oder Alkenylenkette mit jeweils bis zu 6 Kohlenstoffatomen bedeutet, die gegebenenfalls bis zu 2-fach durch Hydroxy substituiert sind,
- R⁷: Wasserstoff, Fluor, Chlor oder Brom bedeutet,
und
- R⁸: Wasserstoff, Fluor, Chlor, Brom, Azido, Trifluormethyl, Hydroxy, Trifluormethoxy, geradkettiges oder verzweigtes Alkoxy mit bis zu 4 Kohlenstoffatomen bedeutet,
oder
- R⁷ und R⁸: mit dem C-Atom eine Carbonylgruppe bilden,
- E: für Cyclopropyl, Cyclobutyl, Cyclopentyl, Cyclohexyl oder Cycloheptyl steht, oder
für geradkettiges oder verzweigtes Alkyl mit bis zu 6 Kohlenstoffatomen steht, das gegebenenfalls durch Cyclopropyl, Cyclobutyl, Cyclopentyl, Cyclohexyl, Cycloheptyl oder durch Hydroxy substituiert ist,
- R¹ und R²: gemeinsam eine geradkettige oder verzweigte Alkylenkette mit bis zu 6 Kohlenstoffatomen bilden, an die ein Phenylring anneliert ist und die durch eine Carbonylgruppe oder eine Gruppe der Formel 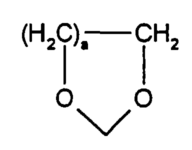 oder -OR¹³ substituiert sein muß,
worin
- a: eine Zahl 1, 2 oder 3 bedeutet
und
- R¹³: Wasserstoff, geradkettiges oder verzweigtes Alkyl, Acyl oder Alkoxycarbonyl mit jeweils bis zu 5 Kohlenstoffatomen oder einen Rest der Formel -SiR¹⁴R¹⁵R¹⁶ bedeutet,
worin
- R¹⁴, R¹⁵ und R¹⁶: gleich oder verschieden sind und
geradkettiges oder verzweigtes Alkyl mit bis zu 5 Kohlenstoffatomen oder Phenyl bedeuten,
und wobei beide Ringsysteme gegebenenfalls bis zu 3-fach gleich oder verschieden durch Fluor, Chlor, Brom, oder durch geradkettiges oder verzweigtes Alkyl oder Alkoxycarbonyl mit jeweils bis zu 5 Kohlenstoffatomen substituiert sind,
und deren Salze und deren N-Oxide, mit Ausnahme der Verbindung 3-Benzoyl-2-methyl-4-phenyl-indeno〈1,2-b〉pyridin-5-on.

Besonders bevorzugt sind Verbindungen der Formel (I)
in welchen
- A: für Phenyl steht, das gegebenenfalls durch Fluor, Chlor, Methyl, Methoxy, Nitro oder Trifluormethyl substituiert ist,
- D: für einen Rest der Formel 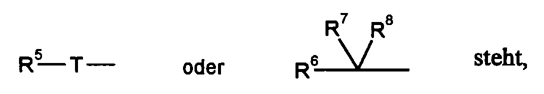 worin
- R⁵ und R⁶: unabhängig voneinander
Cyclopropyl, Phenyl, Pyridyl, Chinolyl, Indolyl, Benzthiazolyl oder Tetrahydronaphthalinyl bedeuten, die gegebenenfalls bis zu 2-fach gleich oder verschieden durch Trifluormethyl, Trifluormethoxy, Fluor, Chlor, Brom, Hydroxy, Carboxyl, durch geradkettiges oder verzweigtes Alkyl, Acyl, Alkoxy oder Alkoxycarbonyl mit jeweils bis zu 4 Kohlenstoffatomen oder durch Phenyl, Phenoxy oder Thiophenyl substituiert sind, die ihrerseits durch Fluor, Chlor, Brom, Trifluormethyl oder Trifluormethoxy substituiert sein können,
- T: eine geradkettige oder verzweigte Alkylen- oder Alkenylenkette mit jeweils bis zu 4 Kohlenstoffatomen bedeutet, die gegebenenfalls bis zu 2-fach durch Hydroxy substituiert sind,
- R⁷: Wasserstoff oder Fluor bedeutet,
und
- R⁸: Wasserstoff, Fluor, Chlor, Brom, Azido, Trifluormethyl, Hydroxy, Trifluormethoxy oder Methoxy bedeutet,
oder
- R⁷ und R⁸: gemeinsam mit dem C-Atom eine Carbonylgruppe bilden,
- E: für Cyclopropyl, Cyclobutyl, Cyclopentyl, Cyclohexyl oder Cycloheptyl steht, oder
für geradkettiges oder verzweigtes Alkyl mit bis zu 5 Kohlenstoffatomen steht, das gegebenenfalls durch Cyclopentyl oder Cyclohexyl substituiert ist,
- R¹ und R²: gemeinsam eine geradkettige oder verzeigte Alkylenkette mit bis zu 5 Kohlenstoffatomen bilden, an die ein Phenylring anneliert ist und die durch eine Carbonylgruppe oder eine Gruppe der Formel 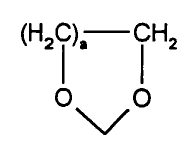 oder -OR¹³ substituiert sein muß,
worin
- a: eine Zahl 1, 2 oder 3 bedeutet
und
- R¹³: Wasserstoff, geradkettiges oder verzweigtes Alkyl, Acyl oder Alkoxycarbonyl mit jeweils bis zu 4 Kohlenstoffatomen oder einen Rest der Formel -SiR¹⁴R¹⁵R¹⁶ bedeutet,
worin
- R¹⁴, R¹⁵ und R¹⁶: gleich oder verschieden sind und
geradkettiges oder verzweigtes Alkyl mit bis zu 4 Kohlenstoffatomen oder Phenyl bedeuten,
und wobei beide Ringsysteme gegebenenfalls bis zu 3-fach gleich oder verschieden durch Fluor, Chlor, geradkettiges oder verzweigtes Alkyl, Alkoxy oder Alkoxycarbonyl mit jeweils bis zu 4 Kohlenstoffatomen substituiert sind,
und deren Salze und N-Oxide, mit Ausnahme der Verbindung 3-Benzoyl-2-methyl-4-phenyl-indeno〈1,2-b〉pyridin-5-on.

Ganz besonders bevorzugt sind erfindungsgemäße Verbindungen der allgemeinen Formel (I),
in welcher
- A: für Phenyl steht, das gegebenenfalls durch Fluor oder Chlor substituiert ist,
- E: für Isopropyl oder Cyclopentyl steht.

Außerdem wurde ein Verfahren zur Herstellung der erfindungsgmäßen Verbindungen der allgemeinen Formel (I) gefunden, dadurch gekennzeichnet, daß man
in Verbindungen der allgemeinen Formel (II) 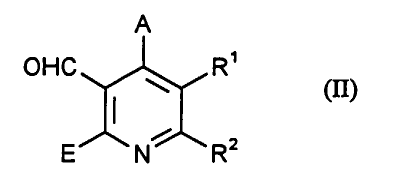 in welcher
- A, E, R¹ und R²: die oben angegebene Bedeutung haben,
zunächst mit metallorganischen Reagenzien im Sinne einer Grignard- oder Wittig-Reaktion den Substituenten D in inerten Lösemitteln einführt,
und gegebenenfalls die unter A, E und/oder R¹ und R² aufgeführten Substituenten nach üblichen Methoden, variiert oder einführt.

Das erfindungsgemäße Verfahren kann durch folgendes Formelschema beispielhaft erläutert werden:

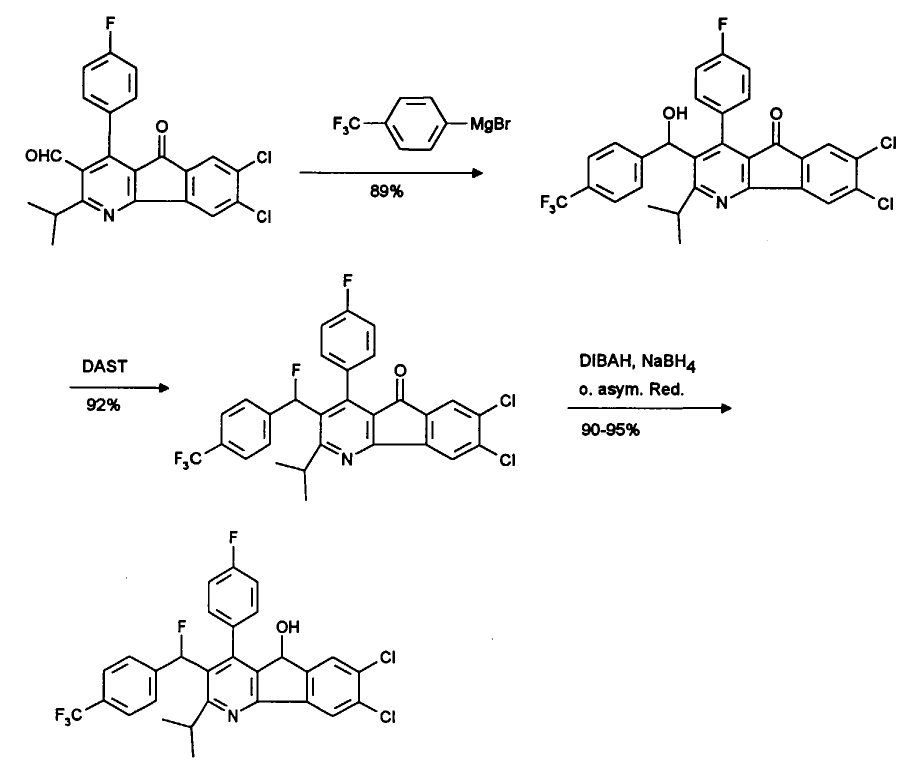

Als Lösemittel eignen sich Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Basen kommen für die einzelnen Schritte die üblichen stark basischen Verbindungen in Frage. Hierzu gehören bevorzugt lithiumorganische Verbindungen wie beispielsweise n-Butyllithium, sec.-Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird n-Butyllithium oder Natriumhydrid eingesetzt.

Als metallorganische Reagenzien eignen sich beispielsweise Systeme wie Mg/Brombenzotrifluorid und p-Trifluormethylphenyllithium.

Als Wittig-Reagenzien eignen sich die üblichen Reagenzien. Bevorzugt ist 3-Trifluormethylbenzyltriphenylphosphoniumbromid.

Als Basen eignen sich im allgemeinen eine der oben aufgeführten Basen, vorzugsweise Li-bis-(triethylbutyl)amid.

Die Base wird in einer Menge von 0,1 mol bis 5 mol, bevorzugt von 0,5 mol bis 2 mol jeweils bezogen auf 1 mol der Ausgangsverbindung eingesetzt.

Die Umsetzung mit Wittig-Reagenzien wird im allgemeinen in einem Temperaturbereich von 0°C bis 150°C, bevorzugt bei 25°C bis 40°C, durchgeführt.

Die Wittig-Reaktionen werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die Reduktionen werden im allgemeinen mit Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid.

Das Reduktionsmittel wird im allgemeinen in einer Menge von 1 mol bis 10 mol, bevorzugt von 1 mol bis 3 mol bezogen auf 1 mol der zu reduzierenden Verbindungen eingesetzt.

Die Reduktion verläuft im allgemeinen in einem Temperaturbereich von -78°C bis +50°C, bevorzugt von -78°C bis 0°C, besonders bevorzugt bei -78°C, im Fall des Diisobutylaluminiumhydrids jeweils in Abhängigkeit von der Wahl des Reduktionsmittels sowie Lösemittel.

Die Reduktion verläuft im allgemeinen bei Normaldruck, es ist aber auch möglich bei erhöhtem oder erniedrigtem Druck zu arbeiten.

Die Hydrierung erfolgt nach üblichen Methoden mit Wasserstoff in Anwesenheit von Edelmetalkatalysatoren, wie beispielsweise Pd/C, Pt/C oder Raney-Nickel in einem der oben aufgeführten Lösemittel, vorzugsweise in Alkoholen wie beispielsweise Methanol, Ethanol oder Propanol, in einem Temperaturbereich von -20°C bis +100°C, bevorzugt von 0°C bis +50°C, bei Normaldruck oder Überdruck.

Als Derivatisierungen seien beispielhaft folgende Reaktionstypen genannt: Oxidationen, Reduktionen, Hydrierungen, Halogenierung, Wittig/Grignard-Reaktionen und Amidierungen/Sulfoamidierungen.

Als Basen kommen für die einzelnen Schritte die üblichen stark basischen Verbindungen in Frage. Hierzu gehören bevorzugt lithiumorganische Verbindungen wie beispielsweise n-Butyllithium, sec.-Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt wird n-Butyllithium oder Natriumhydrid eingesetzt.

Als Basen eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt wird Natrimhydroxid oder Kaliumhydroxid eingesetzt.

Als Lösemittel eignen sich für die einzelnen Reaktionsschritte auch Alkohole wie Methanol, Ethanol, Propanol, Butanol oder tert.Butanol. Bevorzugt ist tert.Butanol.

Gegebenenfalls ist es nötig, einige Reaktionsschritte unter Schutzgasatmosphäre durchzuführen.

Die Halogenierungen erfolgen im allgemeinen in einem der oben aufgeführten chlorierten Kohlenwasserstoffen oder Toluol, wobei Methylenchlorid und Toluol bevorzugt sind.

Als Halogenierungsmittel eignen sich beispielsweise Diethylamino-Schwefeltrifluorid (DAST) oder SOCl₂.

Die Halogenierung verläuft im allgemeinen in einem Temperaturbereich von -78°C bis +50°C, bevorzugt von -78°C bis 0°C, besonders bevorzugt bei -78°C, jeweils in Abhängigkeit von der Wahl des Halogenierungsmittels sowie Lösemittel.

Die Halogenierung verläuft im allgemeinen bei Normaldurck, es ist aber auch möglich bei erhöhtem oder erniedrigtem Druck zu arbeiten.

Die Verbindungen der allgemeinen Formel (II) sind neu und können hergestellt werden, indem man
durch Umsetzung der Verbindungen der allgemeinen Formel (III) 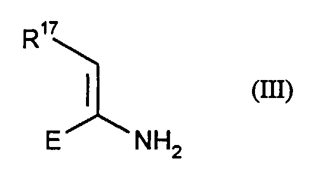 in welcher
- E: die oben angegebene Bedeutung hat
und
- R¹⁷: für C₁-C₄-Alkoxycarbonyl steht,
mit Aldehyden der allgemeinen Formel (IV)

A-CHO (IV)

in welcher
- A: die oben angegebene Bedeutung hat,
und Verbindungen der allgemeinen Formel (V) 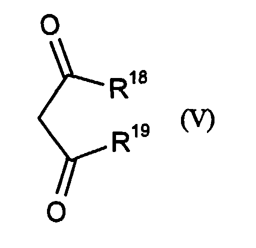 in welcher
- R¹⁸ und R¹⁹: unter Einbezug einer Carbonylgruppe den oben angegebenen Bedeutungsumfang von R¹ und R² umfassen,
die Verbindungen der allgemeinen Formel (VI) 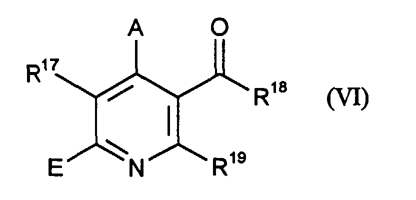 in welcher
- A, E, R¹⁷, R¹⁸ und R¹⁹: die oben angegebene Bedeutung haben,
herstellt,
und in einem letzten Schritt die Alkoxycarbonylgruppe (R¹⁷) durch eine Reduktions-Oxidations-Sequenz in eine Aldehydgruppe überführt.

Als Lösemittel eignen sich für die Oxidation Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Methylenchlorid.

Als Oxidationsmittel eignen sich beispielsweise Schwefeltrioxid-Pyridinkomplex, Cer(IV)-ammoniumnitrat, 2,3-Dichlor-5,6-dicyan-benzochinon, Pyridiniumchlorochromat (PCC), Osmiumtetroxid und Mangandioxid. Bevorzugt ist Schwefeltrioxid-Pyridinkomplex.

Das Oxidationsmittel wird in einer Menge von 1 mol bis 10 mol, bevorzugt von 2 mol bis 5 mol bezogen auf 1 mol der Verbindungen der allgemeinen Formel (IV) eingesetzt.

Die Oxidation verläuft im allgemeinen in einem Temperaturbereich von -50°C bis +100°C, bevorzugt von 0°C bis Raumtemperatur.

Die Oxidation verläuft im allgemeinen bei Normaldruck. Es ist aber auch möglich, die Oxidation bei erhöhtem oder erniedrigtem Druck durchzuführen.

Die Verbindungen der allgemeinen Formel (III), (IV) und (V) sind an sich bekannt oder nach üblichen Methoden herstellbar.

Die Verbindungen der allgemeinen Formel (VI) sind neu und können wie oben beschrieben hergestellt werden.

Die erfindungsgemäßen Verbindungen der allgemeinen Formel (I) haben ein nicht vorhersehbares pharmakologisches Wirkspektrum.

Die erfindungsgemäßen Verbindungen der allgemeinen Formel (I) besitzen wertvolle, im Vergleich zum Stand der Technik überlegene, pharmakologische Eigenschaften, insbesondere sind sie hochwirksame Inhibitoren des Cholesterin-Ester-Transfer-Proteins (CETP) und stimulieren den reversen Cholesterintransport. Die erfindungsgemäßen Wirkstoffe bewirken eine Senkung des LDL-Cholesterinspiegels im Blut bei gleichzeitiger Erhöhung des HDL-Cholesterinspiegels. Sie können deshalb zur Behandlung von Hyperlipoproteinämie, Dyslipidämien, Hypertriglyceridämien, kombinierten Hyperlipidämien oder Arteriosklerose eingesetzt werden.

Die pharmakologische Wirkungen der erfindungsgemäßen Stoffe wurden in folgendem Test bestimmt:

### CETP-Inhibitions-Testung

### Gewinnung von CETP

CETP wird aus humanem Plasma durch Differential-Zentrifugation und Säulenchromatographie in partiell gereinigter Form gewonnen und zum Test verwendet. Dazu wird humanes Plasma mit NaBr auf eine Dichte von 1,21 g pro ml eingestellt und 18 h bei 50.000 Upm bei 4°C zentrifugiert. Die Bodenfraktion (d>1,21 g/ml) wird auf eine Sephadex®Phenyl-Sepharose 4B (Fa. Pharmacia) Säule aufgetragen, mit 0,15 m NaCl/0,001 m TrisHCl pH 7,4 gewaschen und anschließend mit dest. Wasser eluiert. Die CETP-aktiven Fraktionen werden gepoolt, gegen 50mM NaAcetat pH 4,5 dialysiert und auf eine CM-Sepharose® (Fa. Pharmacia)-Säule aufgetragen. Mit einem linearen Gradienten (0-1 M NaCl) wird anschließend eluiert. Die gepoolten CETP-Fraktionen werden gegen 10 mM TrisHCl pH 7,4 dialysiert und anschließend durch Chromatographie über eine Mono Q®-Säule (Fa. Pharmacia) weiter gereinigt.

### Gewinnung von radioaktiv markiertem HDL

50 ml frisches humanes EDTA-Plasma werden mit NaBr auf eine Dichte von 1,12 eingestellt und bei 4°C im Ty 65-Rotor 18 h bei 50.000 Upm zentrifugiert. Die Oberphase wird zur Gewinnung von kaltem LDL verwendet. Die Unterphase wird gegen 3*4 l PDB-Puffer (10 mM Tris/HCl pH 7,4, 0,15 mM NaCl, 1 mM EDTA, 0,02% NaN₃) dialysiert. Pro 10 ml Retentatvolumen wird anschließend 20 µl ³H-Cholesterin (Dupont NET-725; 1 -µC/µl gelöst in Ethanol !) hinzugesetzt und 72 h bei 37°C unter N₂ inkubiert.

Der Ansatz wird dann mit NaBr auf die Dichte 1,21 eingestellt und im Ty 65-Rotor 18 h bei 50.000 Upm bei 20°C zentrifugiert. Man gewinnt die Oberphase und reinigt die Lipoproteinfraktionen durch Gradientenzentrifugation. Dazu wird die isolierte, markierte Lipoproteinfraktion mit NaBr auf eine Dichte von 1,26 eingestellt. Je 4 ml dieser Lösung werden in Zentrifugenröhrchen (SW 40-Rotor) mit 4 ml einer Lösung der Dichte 1,21 sowie 4,5 ml einer Lösung von 1,063 überschichtet (Dichtelösungen aus PDB-Puffer und NaBr) und anschließend 24 h bei 38.000 Upm und 20°C im SW 40-Rotor zentrifugiert. Die zwischen der Dichte 1,063 und 1,21 liegende, das markierte HDL enthaltende Zwischenschicht wird gegen 3*100 Volumen PDB-Puffer bei 4°C dialysiert.
Das Retentat enthält radioaktiv markiertes ³H-CE-HDL, das auf ca. 5x10⁶ cpm pro ml eingestellt zum Test verwendet wird.

### CETP-Test

Zur Testung der CETP-Aktivität wird die Übertragung von ³H-Cholesterolester von humanen HD-Lipoproteinen auf biotinylierte LD-Lipoproteine gemessen.
Die Reaktion wird durch Zugabe von Streptavidin-SPA®beads (Fa. Amersham) beendet und die übertragene Radioaktivität direkt im Liquid Scintillation Counter bestimmt.
Im Testansatz werden 10 µl HDL-³H-Cholesterolester (∼ 50.000 cpm) mit 10 µl Biotin-LDL (Fa. Amersham) in 50 mM Hepes / 0,15 m NaCl / 0,1% Rinderserumalbumin / 0,05% NaN₃ pH 7,4 mit 10 µl CETP (1 mg/ml) und 3 µl Lösung der zu prüfenden Substanz (in 10% DMSO / 1% RSA gelöst), für 18 h bei 37°C inkubiert. Anschließend werden 200 µl der SPA-Streptavidin-Bead-Lösung (TRKQ 7005) zugesetzt, 1 h unter Schütteln weiter inkubiert und anschließend im Scintillationszähler gemessen. Als Kontrollen dienen entsprechende Inkubationen mit 10 µl Puffer, 10 µl CETP bei 4°C sowie 10 µl CETP bei 37°C.
Die in den Kontrollansätzen mit CETP bei 37°C übertragene Aktivität wird als 100% Übertragung gewertet. Die Substanzkonzentration, bei der diese Übertragung auf die Hälfte reduziert ist, wird als IC₅₀-Wert angegeben.

In der folgenden Tabelle A sind die IC₅₀-Werte (mol/l) für CETP-Inhibitoren angegeben:

**Tabelle A**

| **Beispiel-Nr.** | **IC**_{**50**}**-Wert (mol/l)** |
|---|---|
| 14 | 6 x 10⁻⁸ |
| 22 | 2,4 x 10⁻⁷ |
| 27 | 3 x 10⁻⁷ |

### Ex vivo Aktivität der erfindungsgemäßen Verbindungen

Syrische Goldhamster aus werkseigener Zucht werden nach 24-stündigem Fasten narkotisiert (0,8 mg/kg Atropin, 0,8 mg/kg Ketavet® s.c., 30' später 50 mg/kg Nembutal i.p.). Anschließend wird die V.jugularis freipräpariert und kanüliert. Die Testsubstanz wird in einem geeigneten Lösemittel (in der Regel Adalat-Placebolösung: 60 g Glycerin, 100 ml H₂O, ad 1000 ml PEG-400) gelöst und den Tieren über einen in die V.jugularis eingeführten PE-Katheter verabreicht. Die Kontrolltiere erhalten das gleiche Volumen Lösungsmittel ohne Testsubstanz. Anschließend wird die Vene abgebunden und die Wunde verschlossen.
Die Verabreichung der Testsubstanzen kann auch p.o. erfolgen, indem die Substanzen in DMSO gelöst und 0,5% Tylose suspendiert mittels Schlundsonde peroral verabreicht werden. Die Kontrolltiere erhalten identische Volumen Lösemittel ohne Testsubstanz.
Nach verschiedenen Zeitpunkten - bis zu 24 Stunden nach Applikation - wird den Tieren durch Punktion des retro-orbitalen Venenplexus Blut entnommen (ca. 250 µl). Durch Inkubation bei 4°C über Nacht wird die Gerinnung abgeschlossen, anschließend wird 10 Minuten bei 6000 x g zentrifugiert. Im so erhaltenen Serum wird die CETP-Aktivität durch den modifizierten CETP-Test bestimmt. Es wird wie für den CETP-Test oben beschrieben die Übertragung von ³H-Cholesterolester von HD-Lipoproteinen auf biotinylierte LD-Lipoproteine gemessen.
Die Reaktion wird durch Zugabe von Streptavidin-SPA^{R}beads (Fa. Amersham) beendet und die übertragene Radioaktivität direkt im Liquid Scintillation Counter bestimmt.
Der Testansatz wird wie unter "CETP-Test" beschrieben durchgeführt. Lediglich 10 µl CETP werden für die Testung der Serum durch 10 µl der entsprechenden Serumproben ersetzt. Als Kontrollen dienen entsprechende Inkubationen mit Seren von unbehandelten Tieren.
Die in den Kontrollansätzen mit Kontrollseren übertragene Aktivität wird als 100% Übertragung gewertet. Die Substanzkonzentration, bei der diese Übertragung auf die Hälfte reduziert ist wird als ED₅₀-Wert angegeben.

**Tabelle B**

| ED₅₀-Werte für ex vivo-Aktivität | | |
|---|---|---|
| Bsp. | ED₅₀ | % Hemmung bei 10 mg/kg |
| 14 | < 10 mg/kg | 64,0 % |
| 19 | > 10 mg/kg | 46,0 % |
| 29 | > 10 mg/kg | 17,2 % |

### In vivo Aktivität der erfindungsgemäßen Verbindungen

Bei Versuchen zur Bestimmung der oralen Wirkung auf Lipoproteine und Triglyceride wird syrischen Goldhamstern aus werkseigener Zucht Testsubstanz in DMSO gelöst und 0,5% Tylose suspendiert mittels Schlundsonde peroral verabreicht. Zur Bestimmung der CETP-Aktivität wird vor Versuchsbeginn durch retro-orbitale Punktion Blut entnommen (ca. 250 µl). Anschließend werden die Testsubstanzen peroral mittels einer Schlundsonde verabreicht. Die Kontrolltiere erhalten identische Volumen Lösemittel ohne Testsubstanz. Anschließend wird den Tieren das Futter entzogen und zu verschiedenen Zeitpunkten - bis zu 24 Stunden nach Substanzapplikation - durch Punktion des retroorbitalen Venenplexus Blut entnommen.
Durch Inkubation von 4°C über Nacht wird die Gerinnung abgeschlossen, anschließend wird 10 Minunten bei 6000 x g zentrifugiert. Im so erhaltenen Serum wird der Gehalt an Cholesterin und Triglyceriden mit Hilfe modifizierter kommerziell erhältlicher Enzymtests bestimmt (Cholesterin enzymatisch 14366 Merck, Triglyceride 14364 Merck). Serum wird in geeigneter Weise mit physiologischer Kochsalzlösung verdünnt.
100 µl Serum-Verdünnung werden mit 100 µl Testsubstanz in 96-Lochplatten versetzt und 10 Minuten bei Raumtemperatur inkubiert.
Anschließend wird die optische Dichte bei einer Wellenlänge von 492 nm mit einem automatischen Platten-Lesegerät bestimmt. Die in den Proben enthaltene Triglycerid- bzw. Cholesterinkonzentration wird mit Hilfe einer parallel gemessenen Standardkurve bestimmt.
Die Bestimmung des Gehaltes von HDL-Cholesterin wird nach Präzipitation der ApoB-haltigen Lipoproteine mittels eines Reagenziengemisch (Sigma 352-4 HDL Cholesterol Reagenz) nach Herstellerangaben durchgeführt.

**Tabelle C**

| HDL-Anstieg bei in vivo-Versuchen | | |
|---|---|---|
| Bsp. | Dosis (mg/kg) | % HDL-Anstieg |
| 18 | 2 x 10 | 3,3 |
| 19 | 2 x 10 | 17,1 |
| 20 | 2 x 10 | 11,14 |

### In vivo Wirksamkeit an transgenen hCETP-Mäusen

Transgenen Mäusen aus eigener Zucht (Dinchuck, Hart, Gonzalez, Karmann, Schmidt, Wirak; BBA (1995), 1295, 301) wurden die zu prüfenden Substanzen im Futter verabreicht. Vor Versuchsbeginn wurde den Mäusen retroorbital Blut entnommen, um Cholesterin und Triglyceride im Serum zu bestimmen. Das Serum wurde wie oben für Hamster beschrieben durch Inkubation bei 4°C über Nacht und anschließender Zentrifugation bei 6000 x g gewonnen. Nach einer Woche wurde den Mäusen wieder Blut entnommen, um Lipoproteine und Triglyceride zu bestimmen. Die Veränderung der gemessenen Parameter wird als prozentuale Veränderung gegenüber dem Ausgangswert ausgedrückt.

**Tabelle D**

| Bsp. | HDL | LDL | Triglyceride |
|---|---|---|---|
| 14 (100 ppm) | 22,0 % | - 10,5 % | - 9,7 % |

Die Erfindung betrifft außerdem die Kombination von biyclisch annellierten Pyridinen der allgemeinen Formel (I) mit einem Glucosidase- und/oder Amylasehemmer zur Behandlung von familiärer Hyperlipidaeamien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Weiterhin können die erfindungsgemäßen Verbindungen in Kombination mit Cholesterin senkenden Vastatinen oder ApoB-senkenden Prinzipien verwendet werden, um Dyslipidämien, kombinierte Hyperlipidämien, Hypercholesterolämien oder Hypertriglyceridämien zu behandeln.

Die genannten Kombinationen sind auch zur primären oder sekundären Prävention der koronaren Herzerkrankung einsetzbar.

Vastatine im Rahmen der Erfindung sind beispielsweise Lovastatin, Simvastatin, Pravastatin, Fluvastatin, Atorvastatin und Cerivastatin. Apo B-senkende Mittel sind zum Beispiel MTP-Inhibitoren.

Bevorzugt ist die Kombination von Cerivastatin oder Apo B-Inhibitoren mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösermitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise intravenös, parenteral, perlingual oder vorzugsweise oral.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Verwendete Abkürzungen:

- C =: Cyclohexan
- EE =: Essigester
- PE =: Petrolether
- THF =: Tetrahydrofuran

### Ausgangsverbindungen

### Beispiel I

### 7,8-Dichlor-4-(4-fluor-phenyl)-2-isopropyl-5-oxo-5H-indeno[1,2-b]pyridin-3-carbonsäuremethylester

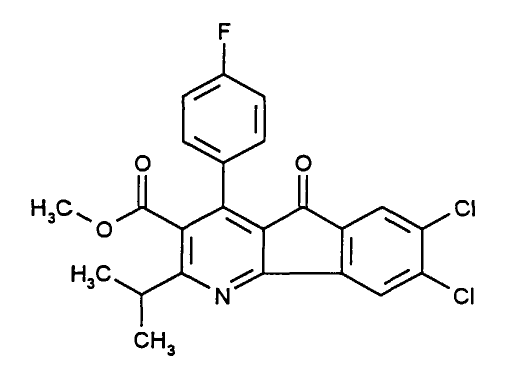

In 300 ml Toluol werden 44 g 5,6-Dichlor-1,3-indandion (204 mmol), 29,2 g 3-Amino-4-methyl-2-pentensäuremethylester (204 mmol) und 25,3 g p-Fluorbenzaldehyd (204 mmol) gelöst und am Wasserabscheider auf Rückflußtemperatur für 18 h erhitzt. Nach Abkühlen wird abfiltriert und das Filtrat eingeengt. Das Rohprodukt wird an 1,2 kg Kieselgel (0,04 - 0,063 mm) mit Cyclohexan : Essigester 9:1 eluiert.
Ausbeute: 14,5 g (16 % d. Th.)
R_{f} = 0,37 (C/EE 9:1)

### Beispiel II

### 7,8-Dichlor-4-(4-fluorphenyl)-3-hydroxymethyl-2-isopropyl-5H-indeno[1,2-b]pyridin-5-ol

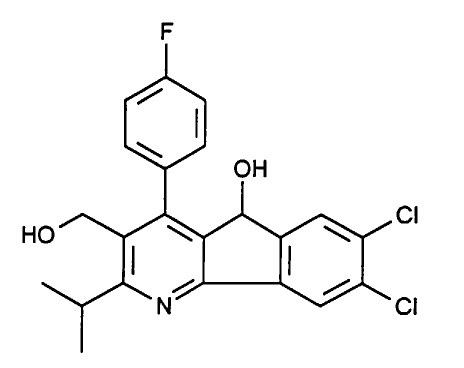

Unter Argon werden 7,4 g der Verbindung aus Beispiel I (16,7 mmol) in 155 ml absolutem Toluol gelöst und bei -70°C 66,6 ml 1 molare Diisobutylaluminiumhydridlösung in Toluol (66,6 mmol) zugetropft. Nach 30 min Rühren bei -70°C und 30 min bei -60°C werden langsam 15,6 ml Methanol zugetropft und das Kühlbad entfernt. Folgend wird mit 100 ml Toluol verdünnt und 900 ml 20 %ige Natrium-Kalium-tartrat-Lösung zugegeben und für 1 h gerührt. Nach Trennung der organischen und wässrigen Phasen wird die wässrige Phase mit Toluol nachextrahiert, die vereinigten organischen Phasen mit gesättigter Natriumchloridlösung gewaschen und darauf über Natriumsulfat getrocknet. Das aus der organischen Phase eingeengte Rohprodukt wird an 600g Kieselgel (0,04 - 0,063 mm) mit C/EE 8:2 eluiert.
Ausbeute: 4,47 g (64 % d. Th.)
R_{f} = 0,12 (C/EE 8:2)

### Beispiel III

### 7,8-Dichlor-4-(fluorphenyl)-2-isopropyl-5-oxo-5H-indeno[1,2-b]pyridin-3-carbaldehyd

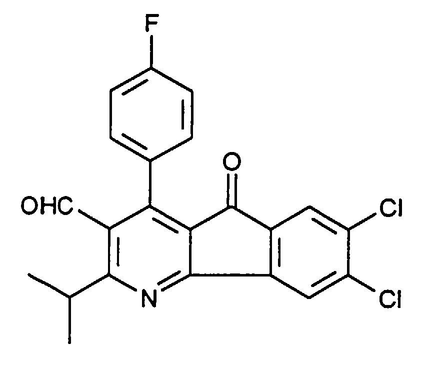

Zu einer Lösung von 2,1 g aus Beispiel II (5 mmol) in 140 ml Methylenchlorid wird bei Raumtemperatur eine Mischung aus 6,8 g Pyridiniumchlorochromat (30 mmol) und 3,2 g Aluminiumoxid (30 mmol) gegeben. Nach 1,5 h wird etwas Kieselgel zugesetzt, über 280 g Kieselgel abgesaugt und mit ca. 2000 ml Methylenchlorid nachgewaschen. Die vereinigten Filtrate werden eingeengt und im Hochvakuum getrocknet.
Ausbeute: 1,8 g (87 % d. Th.)
R_{f} = 0,45 (C/EE 8:2)

### Herstellungsbeispiele

### Beispiel 1

### 7,8-Dichlor-4-(4-fluorphenyl)-3-[hydroxy-(4-trifluormethyl-phenyl)-methyl]-2-isopropyl-indeno[1,2-b]pyridin-5-on

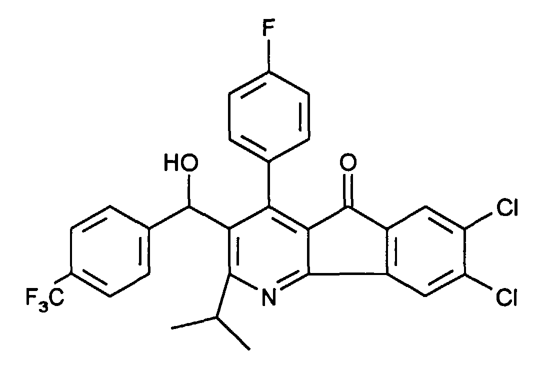

a) Darstellung des Grignard-Reagenzes
   Unter Argon werden zu 537 mg Magnesium (22,4 mmol) 100 ml THF gegeben, wenige Tropfen Dibrommethan zugesetzt und auf Rückfluß erhitzt. Zu der refluxierenden Suspension werden langsam 3,47 g p-Brombenzotrifluorid (15,6 mmol), gelöst in 20 ml THF, zugetropft. Nach 2 h wird auf Raumtemperatur abgekühlt.
b) Grignardaddition an Substanz aus Beispiel III
   Zu einer Lösung von 2,18 g aus Beispiel III (5,2 mmol) in 50 ml THF wird bei -20° C die unter a) synthetisierte Grignardlösung zugetropft. Nach 30 min werden 50 ml gesättigte Ammoniumchloridlösung zugegeben und 10 min gerührt. Es wird mit Wasser und Toluol verdünnt, die Phasen getrennt, die wässrige Phase mit Toluol nachextrahiert, die vereinigten organischen Phasen mit gesättigter Natriumchloridlösung gewaschen und zuletzt die organische Phase über Natriumsulfat getrocknet und eingeengt. Das Rohprodukt wird an 600g Kieselgel (0,04 - 0,063 mm) mit C/EE 9:1 eluiert. Die Eluate werden kristallisiert.
   Ausbeute: 2,6 g (89 % d. Th.)
   R_{f} = 0,32 (C/EE 8:2)

### Beispiel 2

### 7,8-Dichlor-4-(4-fluorphenyl)-3-[fluoro-(4-trifluormethyl-phenyl)-methyl]-2-isopropyl-indeno[1,2-b]pyridin-5-on

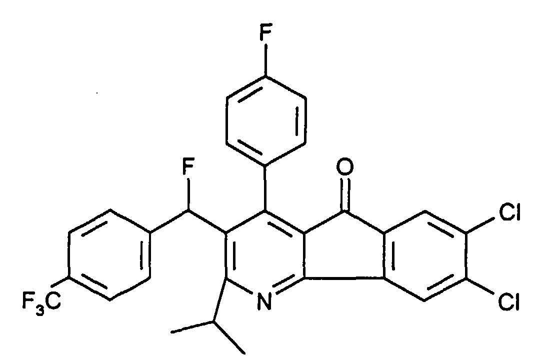

In 10 ml Methylenchlorid werden unter Argonatmosphäre 260 mg aus Beispiel 1 (0,46 mmol) gelöst und bei -70° C 0,06 ml Diethylaminoschwefeltrifluorid in 1 ml Methylenchlorid zugetropft. Nach 10 min bei dieser Temperatur werden 6 ml gesättigte Natriumhydrogencarbonatlösung zugegeben und auf Raumtemperatur erwärmt. Die Phasen werden getrennt, die organische Phase nochmals mit Wasser gewaschen, über Natriumsulfat getrocknet, filtriert und eingeengt. An 20 g Kieselgel (0,04 - 0,063 mm) wird mit C/EE 95:5 eluiert.
Ausbeute: 184 mg (71 % d. Th.)
R_{f} = 0,43 (C/EE 9:1)

### Beispiel 3

### 7,8-Dichlor-4-(4-fluorphenyl)-3-[fluor-(4-trifluormethyl-phenyl)-methyl]-2-isopropyl-5H-indeno[1,2-b]pyridin-5-ol

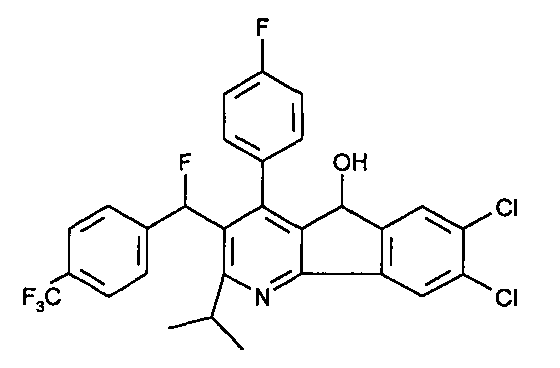

Zu einer Lösung von 122 mg aus Beispiel 2 (0,2 mmol) in 10 ml Methanol werden bei 0° C 16 mg Natriumborhydrid (0,4 mmol) zugesetzt und 2 h bei Raumtemperatur nachgerührt. Es wird mit gesättigter Ammoniumchloridlösung versetzt und dreimal mit Toluol extrahiert. Die vereinigten organischen Phasen werden über Natriumsulfat getrocknet, filtriert und eingeengt. Das Rohprodukt wird an Kieselgel (0,04 - 0,063 mm) mit C/EE 97:3 eluiert.
Ausbeute: 119 mg (97 % d. Th.)
R_{f} = 0,17 (C/EE 9:1)

### Trennung der Diastereomere:

Mittels präparativer HPLC (250 x 25 mm, RP18, 7 µm; Flußrate 6 ml/min mit Acetonitril / Wasser 8:2).
110 mg aus der Verbindung Beispiel VI werden in 2 Injects über die Säule getrennt.
Ausbeute: 56 mg Diastereomer A, 41 mg Diastereomer B
R_{f} = 0,17 (C/EE 9:1)

In Analogie zu den Vorschriften der Beispiele 1-3 werden die in Tabelle 1 aufgeführten Verbindungen hergestellt:

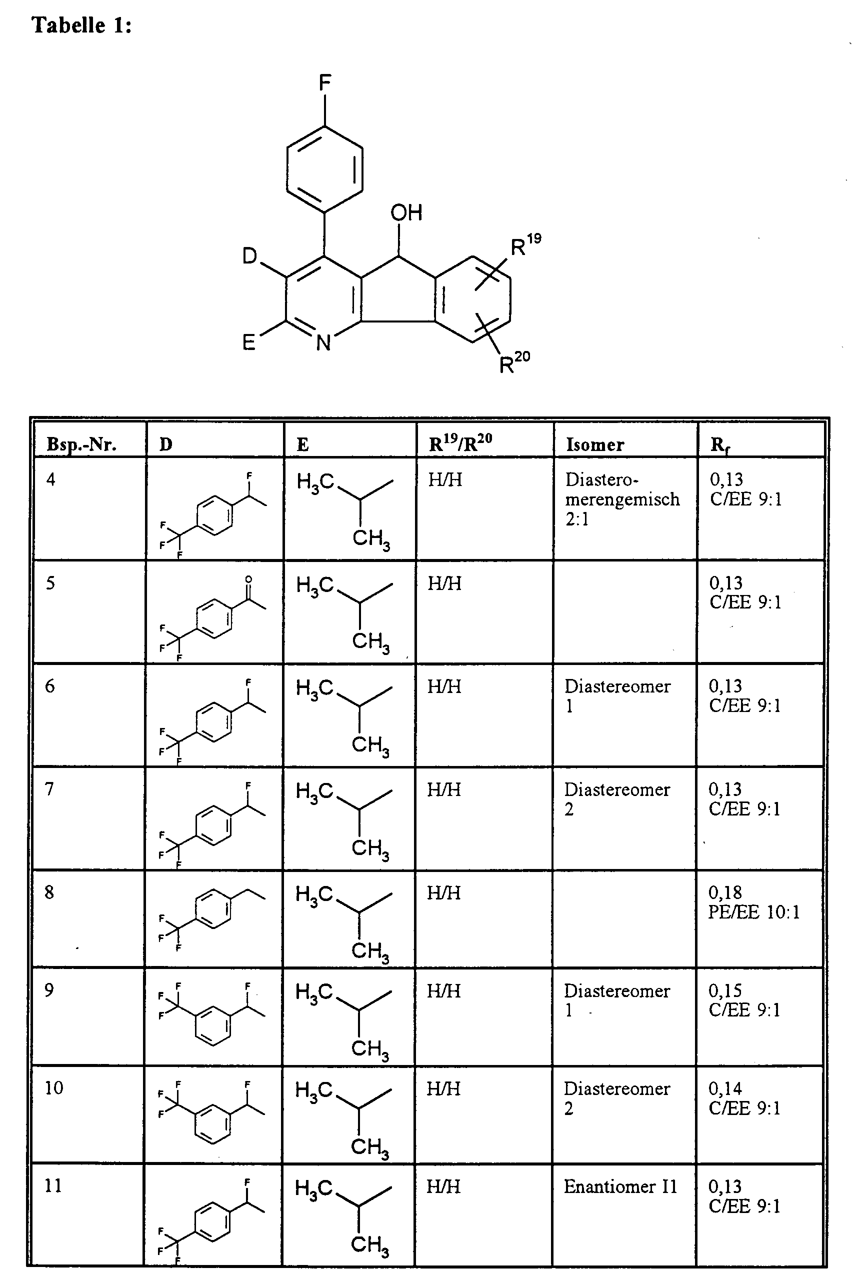

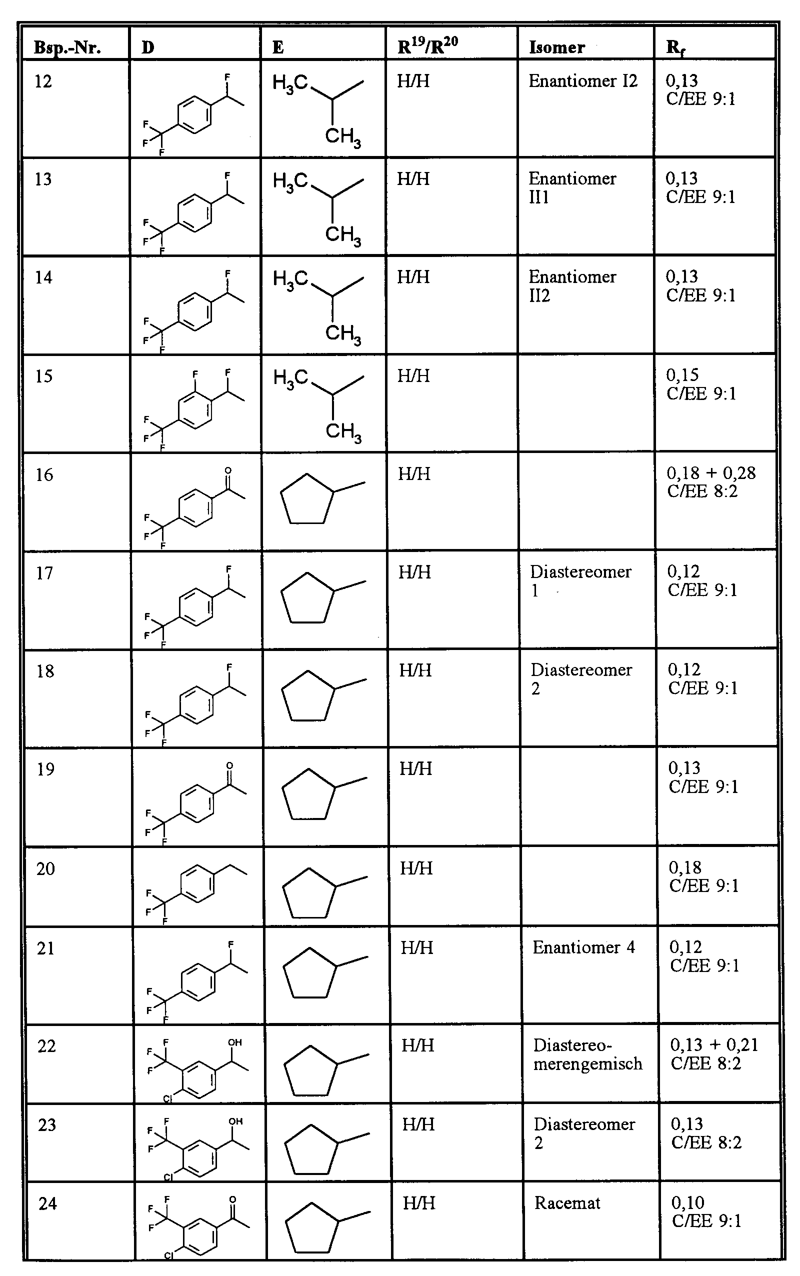

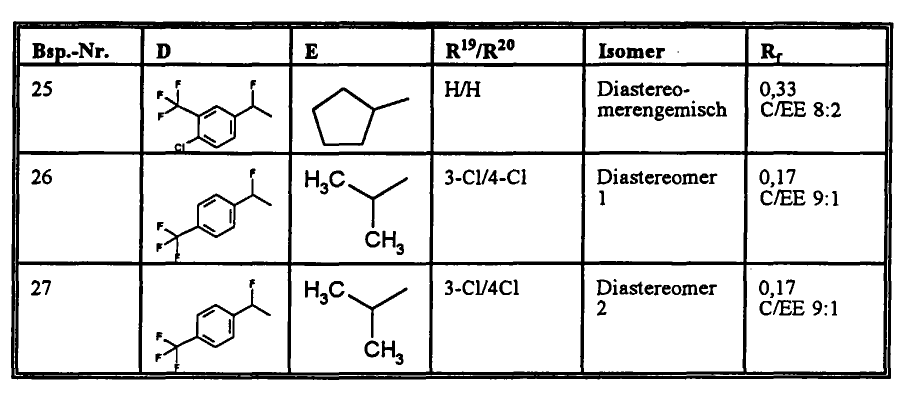

In Analogie zu den oben aufgeführten Vorschriften werden die in der Tabelle 2 aufgeführten Verbindungen hergestellt:

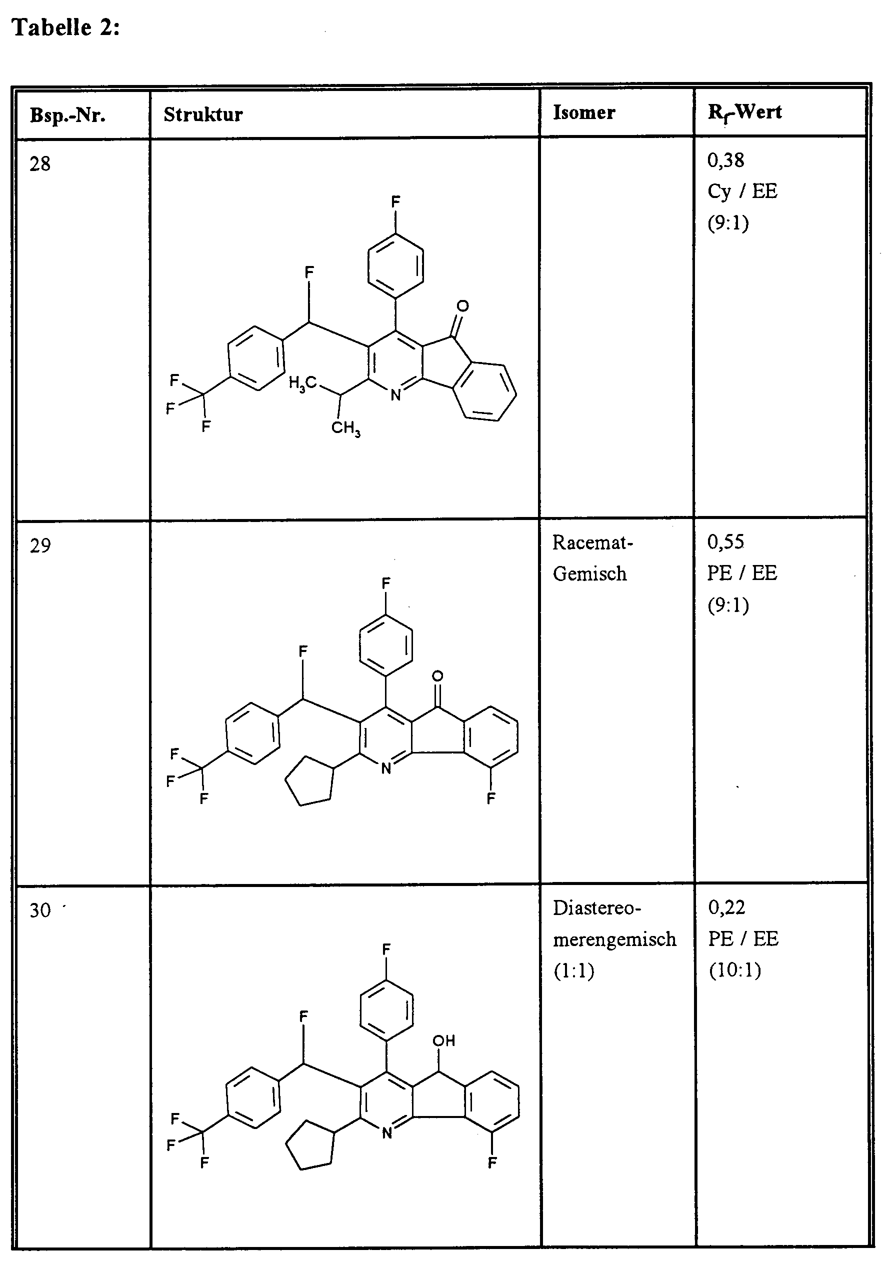

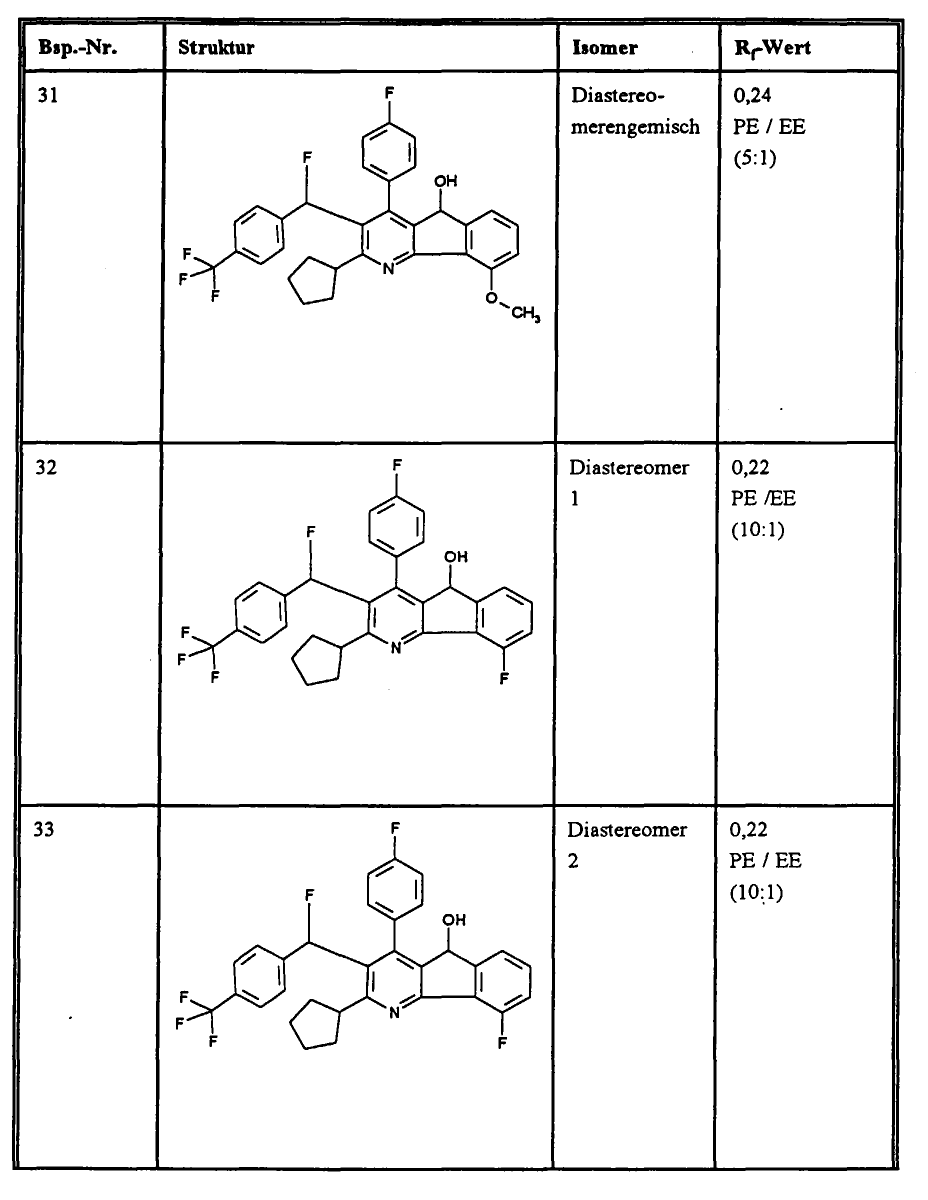

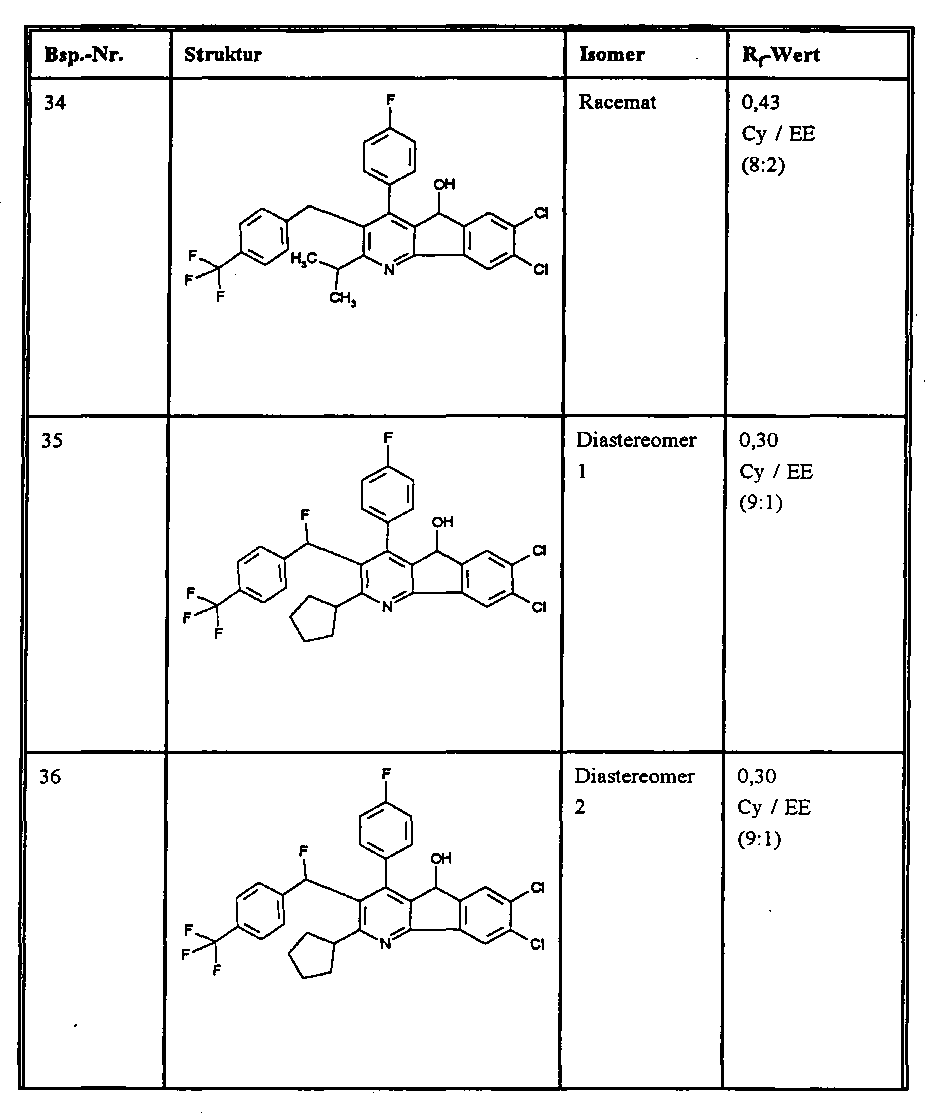

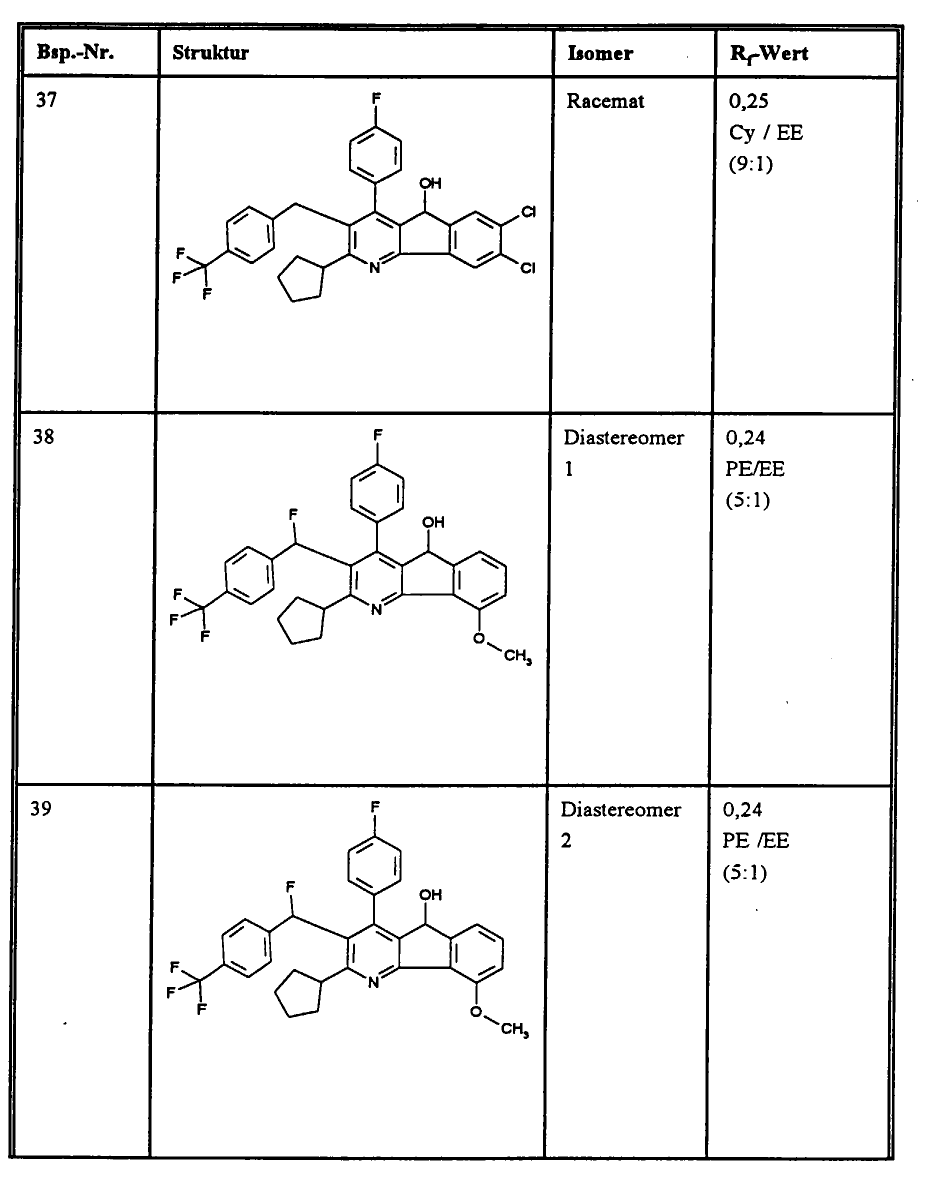

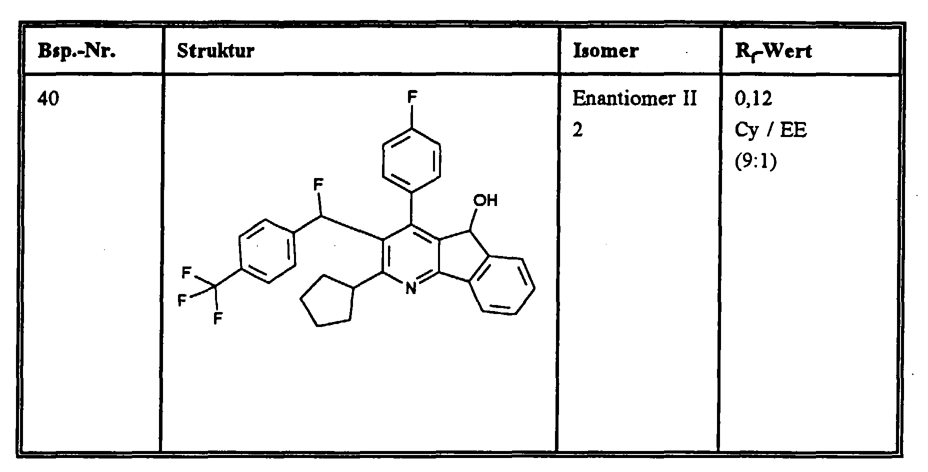

## Patentansprüche

1. Bicyclisch kondensierte Pyridine der allgemeinen Formel (I) 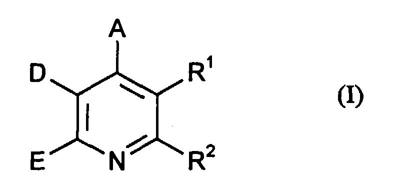 in welcher
A für Aryl mit 6 bis 10 Kohlenstoffatomen steht, das gegebenenfalls bis zu 5-fach gleich oder verschieden durch Halogen, Hydroxy, Trifluormethyl, Trifluormethoxy, Nitro oder durch geradkettiges oder verzweigtes Alkyl, Acyl, Hydroxyalkyl oder Alkoxy mit jeweils bis zu 7 Kohlenstoffatomen, oder durch eine Gruppe der Formel -NR³R⁴ substituiert ist,
worin
R³ und R⁴ gleich oder verschieden sind und
Wasserstoff, Phenyl oder geradkettiges oder verzweigtes Alkyl mit bis zu 6 Kohlenstoffatomen bedeuten,
D für einen Rest der Formel 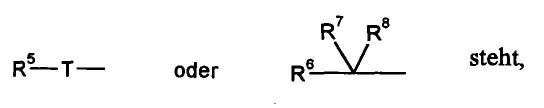 worin
R⁵ und R⁶ unabhängig voneinander
Cycloalkyl mit 3 bis 8 Kohlenstoffatomen bedeuten, oder
Aryl mit 6 bis 10 Kohlenstoffatomen,
oder einen 5- bis 7-gliedrigen aromatischen, gegebenenfalls benzokondensierten Heterocyclus mit bis zu 3 Heteroatomen aus der Reihe S, N und/oder O bedeuten, die gegebenenfalls bis zu 5-fach gleich oder verschieden durch Trifluormethyl, Trifluormethoxy, Halogen, Hydroxy, Carboxyl, durch geradkettiges oder verzweigtes Alkyl, Acyl, Alkoxy oder Alkoxycarbonyl mit jeweils bis zu 6 Kohlenstoffatomen oder durch Phenyl, Phenoxy oder Thiophenyl substituiert sind, die ihrerseits durch Halogen, Trifluormethyl oder Trifluormethoxy substituiert sein können,
oder die Cyclen durch eine Gruppe der Formel -NR⁹R¹⁰ substituiert sind,
worin
R⁹ und R¹⁰ gleich oder verschieden sind und die oben angegebene Bedeutung von R³ und R⁴ haben,
T eine geradkettige oder verzweigte Alkylen- oder Alkenylenkette mit jeweils bis zu 10 Kohlenstoffatomen bedeutet, die gegebenenfalls bis zu 2-fach durch Hydroxy substituiert sind,
R⁷ Wasserstoff oder Halogen bedeutet,
und
R⁸ Wasserstoff, Halogen, Azido, Trifluormethyl, Hydroxy, Trifluormethoxy, geradkettiges oder verzweigtes Alkoxy mit bis zu 5 Kohlenstoffatomen oder einen Rest der Formel -NR¹¹R¹² bedeutet,
worin
R¹¹ und R¹² gleich oder verschieden sind und die oben angegebene Bedeutung von R³ und R⁴ haben,
oder
R⁷ und R⁸ gemeinsam mit dem C-Atom eine Carbonylgruppe bilden,
E für Cycloalkyl mit 3 bis 8 Kohlenstoffatomen steht, oder
für geradkettiges oder verzweigtes Alkyl mit bis zu 8 Kohlenstoffatomen steht, das gegebenenfalls durch Cycloalkyl mit 3 bis 8 Kohlenstoffatomen oder durch Hydroxy substituiert ist,
R¹ und R² gemeinsam eine geradkettige oder verzweigte Alkylenkette mit bis zu 8 Kohlenstoffatomen bilden, an die ein Phenylring anneliert ist und die durch eine Carbonylgruppe oder eine Gruppe der Formel 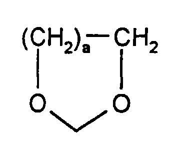 oder -OR¹³ substituiert sein muß,
worin
a für eine Zahl 1, 2 oder 3 steht,
und
R¹³ Wasserstoff geradkettiges oder verzweigtes Alkyl, Acyl oder Alkoxycarbonyl mit jeweils bis zu 6 Kohlenstoffatomen oder einen Rest der Formel -SiR¹⁴R¹⁵R¹⁶ bedeutet,
worin
R¹⁴, R¹⁵ und R¹⁶ gleich oder verschieden sind und
geradkettiges oder verzweigtes Alkyl mit bis zu 6 Kohlenstoffatomen oder Phenyl bedeuten,
und wobei beide Ringsysteme gegebenenfalls bis zu 3-fach gleich oder verschieden durch Halogen, Carboxyl oder durch geradkettiges oder verzweigtes Alkyl, Alkoxy oder Alkoxycarbonyl mit jeweils bis zu 6 Kohlenstoffatomen substituiert sind,
und deren Salze und deren N-Oxide.

2. Bicyclisch kondensierte Pyrindine der Formel nach Anspruch 1
in welcher
A für Naphthyl oder Phenyl steht, die gegebenenfalls bis zu 3-fach gleich oder verschieden durch Fluor, Chlor, Brom, Hydroxy, Trifluormethyl, Trifluormethoxy, Nitro oder durch geradkettiges oder verzweigtes Alkyl oder Alkoxy mit jeweils bis zu 6 Kohlenstoffatomen substituiert sind,
D für einen Rest der Formel 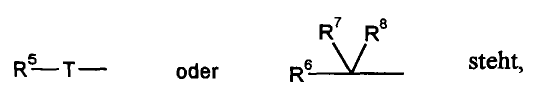 worin
R⁵ und R⁶ unabhängig voneinander
Cyclopropyl, Cyclopentyl oder Cyclohexyl bedeuten, oder Naphthyl, Phenyl, Pyridyl, Chinolyl, Indolyl, Benzthiazolyl oder Tetrahydronaphthalinyl bedeuten, die gegebenenfalls bis zu 3-fach gleich oder verschieden durch Trifluormethyl, Trifluormethoxy, Fluor, Chlor, Brom, Hydroxy, Carboxyl, durch geradkettiges oder verzweigtes Alkyl, Acyl, Alkoxy oder Alkoxycarbonyl mit jeweils bis zu 5 Kohlenstoffatomen oder durch Phenyl, Phenoxy oder Thiophenyl substituiert sind, die ihrerseits durch Fluor, Chlor, Brom, Trifluormethyl oder Trifluormethoxy substituiert sein können,
T eine geradkettige oder verzweigte Alkylen- oder Alkenylenkette mit jeweils bis zu 6 Kohlenstoffatomen bedeutet, die gegebenenfalls bis zu 2-fach durch Hydroxy substituiert sind,
R⁷ Wasserstoff, Fluor, Chlor oder Brom bedeutet,
und
R⁸ Wasserstoff, Fluor, Chlor, Brom, Azido, Trifluormethyl, Hydroxy, Trifluormethoxy, geradkettiges oder verzweigtes Alkoxy mit bis zu 4 Kohlenstoffatomen bedeutet,
oder
R⁷ und R⁸ mit dem C-Atom eine Carbonylgruppe bilden,
E für Cyclopropyl, Cyclobutyl, Cyclopentyl, Cyclohexyl oder Cycloheptyl steht, oder
für geradkettiges oder verzweigtes Alkyl mit bis zu 6 Kohlenstoffatomen steht, das gegebenenfalls durch Cyclopropyl, Cyclobutyl, Cyclopentyl, Cyclohexyl, Cycloheptyl oder durch Hydroxy substituiert ist,
R¹ und R² gemeinsam eine geradkettige oder verzweigte Alkylenkette mit bis zu 6 Kohlenstoffatomen bilden, an die ein Phenylring anneliert ist und die durch eine Carbonylgruppe oder eine Gruppe der Formel 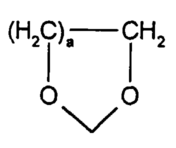 oder -OR¹³ substituiert sein muß,
worin
a eine Zahl 1, 2 oder 3 bedeutet
und
R¹³ Wasserstoff, geradkettiges oder verzweigtes Alkyl, Acyl oder Alkoxycarbonyl mit jeweils bis zu 5 Kohlenstoffatomen oder einen Rest der Formel -SiR¹⁴R¹⁵R¹⁶ bedeutet,
worin
R¹⁴, R¹⁵ und R¹⁶ gleich oder verschieden sind und
geradkettiges oder verzweigtes Alkyl mit bis zu 5 Kohlenstoffatomen oder Phenyl bedeuten,
und wobei beide Ringsysteme gegebenenfalls bis zu 3-fach gleich oder verschieden durch Fluor, Chlor, Brom, oder durch geradkettiges oder verzweigtes Alkyl oder Alkoxycarbonyl mit jeweils bis zu 5 Kohlenstoffatomen substituiert sind,
und deren Salze und deren N-Oxide.

3. Bicyclisch kondensierte Pyridine der Formel nach Anspruch 1
in welchen
A für Phenyl steht, das gegebenenfalls durch Fluor, Chlor, Methyl, Nitro, Methoxy oder Trifluormethyl substituiert ist,
D für einen Rest der Formel 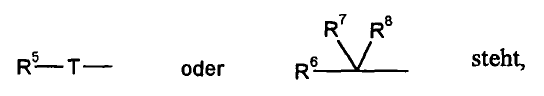 worin
R⁵ und R⁶ unabhängig voneinander
Cyclopropyl, Phenyl, Pyridyl, Chinolyl, Indolyl, Benzthiazolyl oder Tetrahydronaphthalinyl bedeuten, die gegebenenfalls bis zu 2-fach gleich oder verschieden durch Trifluormethyl, Trifluormethoxy, Fluor, Chlor, Brom, Hydroxy, Carboxyl, durch geradkettiges oder verzweigtes Alkyl, Acyl, Alkoxy oder Alkoxycarbonyl mit jeweils bis zu 4 Kohlenstoffatomen oder durch Phenyl, Phenoxy oder Thiophenyl substituiert sind, die ihrerseits durch Fluor, Chlor, Brom, Trifluormethyl oder Trifluormethoxy substituiert sein können,
T eine geradkettige oder verzweigte Alkylen- oder Alkenylenkette mit jeweils bis zu 4 Kohlenstoffatomen bedeutet, die gegebenenfalls bis zu 2-fach durch Hydroxy substituiert sind,
R⁷ Wasserstoff oder Fluor bedeutet,
und
R⁸ Wasserstoff, Fluor, Chlor, Brom, Azido, Trifluormethyl, Hydroxy, Trifluormethoxy oder Methoxy bedeutet,
oder
R⁷ und R⁸ gemeinsam mit dem C-Atom eine Carbonylgruppe bilden,
E für Cyclopropyl, Cyclobutyl, Cyclopentyl, Cyclohexyl oder Cycloheptyl steht, oder
für geradkettiges oder verzweigtes Alkyl mit bis zu 5 Kohlenstoffatomen steht, das gegebenenfalls durch Cyclopentyl oder Cyclohexyl substituiert ist,
R¹ und R² gemeinsam eine geradkettige oder verzeigte Alkylenkette mit bis zu 5 Kohlenstoffatomen bilden, an die ein Phenylring anneliert ist und die durch eine Carbonylgruppe oder eine Gruppe der Formel 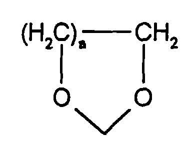 oder -OR¹³ substituiert sein muß,
worin
a eine Zahl 1, 2 oder 3 bedeutet
und
R¹³ Wasserstoff, geradkettiges oder verzweigtes Alkyl, Acyl oder Alkoxycarbonyl mit jeweils bis zu 4 Kohlenstoffatomen oder einen Rest der Formel -SiR¹⁴R¹⁵R¹⁶ bedeutet,
worin
R¹⁴, R¹⁵ und R¹⁶ gleich oder verschieden sind und
geradkettiges oder verzweigtes Alkyl mit bis zu 4 Kohlenstoffatomen oder Phenyl bedeuten,
und wobei beide Ringsysteme gegebenenfalls bis zu 3-fach gleich oder verschieden durch Fluor, Chlor, geradkettiges oder verzweigtes Alkyl, Alkoxy oder Alkoxycarbonyl mit jeweils bis zu 4 Kohlenstoffatomen substituiert sind,
und deren Salze und N-Oxide.

4. Bicyclisch kondensierte Pyridine nach Anspruch 1 bis 3 als Arzneimittel.

5. Verfahren zur Herstellung von bicyclisch kondiserten Pyridinen nach Anspruch 1 bis 3, dadurch gekennzeichnet, daß man
in Verbindungen der allgemeinen Formel (II) 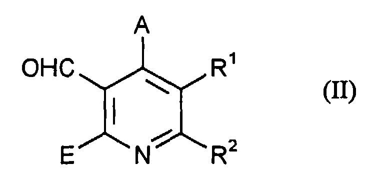 in welcher
A, E, R¹ und R² die oben angegebene Bedeutung haben,
zunächst mit metallorganischen Reagenzien im Sinne einer Grignard- oder Wittig-Reaktion den Substituenten D in inerten Lösemitteln einführt,
und gegebenenfalls die unter A, E und/oder R¹ und R² aufgeführten Substituenten nach üblichen Methoden, variiert oder einführt.

6. Arzneimittel enthaltend mindestens ein bicyclisch kondensiertes Pyridin nach Anspruch 1 bis 3.

7. Arzneimittel nach Anspruch 6 zur Behandlung von Hyperlipopropteinaemia.

8. Arzneimittel nach Anspruch 7 zur Behandlung von Arteriosklerose.

9. Verwendung von bicyclisch kondensierten Pyridinen nach Anspruch 1 bis 3 zur Herstellung von Arzneimitteln.

10. Verwendung nach Anspruch 9 zur Herstellung von Arzneimitteln zur Behandlung von Arteriosklerose.
